# EUROPEAN PATENT SPECIFICATION

(11) **EP 3 943 060 B1**
(45) Date of publication and mention of the grant of the patent: **21.05.2025**
(21) Application number: 20776620.5
(22) Date of filing: 05.03.2020
(51) Int. Cl.: A61J 15/00, A61M 25/00, A61M 39/16, A61B 90/70, A61F 13/38

(54) **WIPING TIP**
WISCHBLATTSPITZE
POINTE D'ESSUYAGE

(30) Priority: 22.03.2019 JP 2019054556
(43) Date of publication of application: 26.01.2022
(73) Proprietor: JMS Co., Ltd., Hiroshima-shi, Hiroshima 730-8652 (JP)
(72) Inventor: UKITA, Junji, Hiroshima 730-8652 (JP); UEHARA, Yasumasa, Hiroshima 730-8652 (JP); WADA, Ryohei, Hiroshima 730-8652 (JP)
(74) Representative: Dehns
(86) International application number: PCT/JP2020/009511
(87) International publication number: WO 2020/195689

(56) References cited:
- WO-A1-2016/179368
- WO-A1-2017/094873
- CN-A- 108 743 996
- CN-A- 108 743 996
- JP-A- 2006 320 430
- JP-A- 2009 100 893
- JP-A- 2009 100 893
- JP-A- 2012 522 593
- JP-A- 2017 099 738
- JP-A- H10 108 832
- JP-A- H10 108 832
- JP-A- H10 113 328
- JP-U- S5 391 288
- JP-U- S5 391 288
- US-A1- 2014 322 075
- US-B1- 6 641 551
- US-B1- 6 641 551

## Description

### Technical Field

The present invention relates to a wiping tip for cleaning a male connector that is preferably used for enteral nutrition.

### Background Art

Enteral nutrition is known as a method for administering liquid matter containing nutrient agents, drugs, or the like to patients who cannot use their mouth to eat. In enteral nutrition, a catheter is inserted from outside the body into the gastrointestinal tract (e.g., the stomach) of a patient and is indwelled therein. Known examples of the catheter include a nasal catheter, which is inserted from the nose of a patient, and a PEG (percutaneous endoscopic gastrostomy) catheter, which is inserted into a gastrostomy formed in the abdomen of a patient. Liquid matter such as nutrient agents, liquid foods (commonly referred to as "enteral nutrients"), or drugs is administered to a patient via a catheter. A container storing liquid matter is connected to a catheter (a nasal catheter, a PEG catheter, etc.) indwelled in a patient. In order to connect different members, a connector constituted by a male connector and a female connector is used.

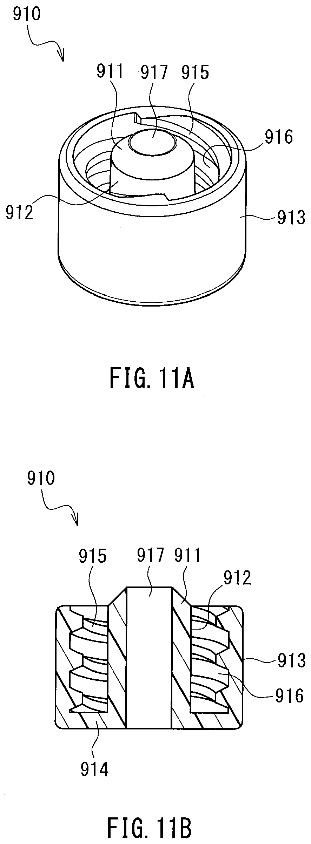

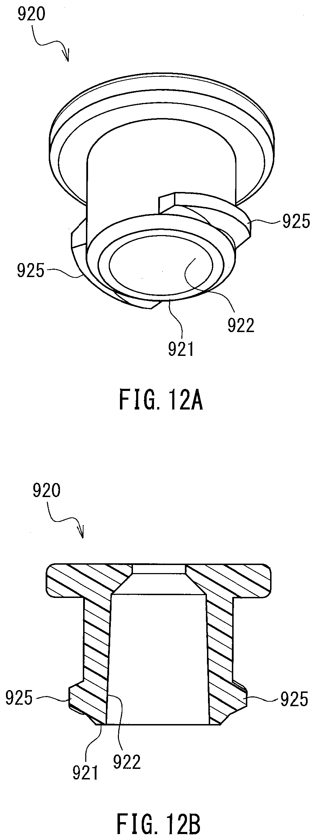

As this sort of connector, a connector including a male connector 910 shown in FIGS. 11A and 11B and a female connector 920 shown in FIGS. 12A and 12B is used.

The male connector 910 in FIGS. 11A and 11B has a cylindrical male member 911 and an outer cylinder 913 that surrounds the male member 911. The male member 911 and the outer cylinder 913 are connected to each other via a bottom plate 914 protruding in a flange shape along the radial direction from the base end of the male member 911. An outer circumferential face 912 of the male member 911 is a tapered surface (a so-called male tapered surface) whose outer diameter decreases toward an end thereof. The male member 911 is provided with a flow path 917 that extends through the male member 911 along the longitudinal direction thereof. An inner circumferential face of the outer cylinder 913 facing the male member 911 is provided with a female thread 915. The female thread 915 is a right-hand thread.

The female connector 920 in FIGS. 12A and 12B has a hollow cylindrical tubular portion (female member) 921. An inner circumferential face 922 of the tubular portion 921 is a tapered surface (a so-called female tapered surface) whose inner diameter increases toward an end thereof. An outer circumferential face of the tubular portion 921 is provided with a spiral protrusion (male thread) 925.

The male connector 910 and the female connector 920 are connected by inserting the male member 911 into the tubular portion 921 and screwing the female thread 915 and the spiral protrusion 925 to each other. The outer circumferential face 912 of the male member 911 and the inner circumferential face 922 of the tubular portion 921 are tapered surfaces with the same diameter and taper angle, and thus they are in liquid-tight surface contact.

In enteral nutrition, the female connector 920 is used as a connector on the upstream side (container side) and the male connector 910 is used as a connector on the downstream side (patient side) with respect to the flow direction of liquid matter.

In the male connector 910, the outer cylinder 913 surrounds the male member 911, and the inner circumferential face of the outer cylinder 913 is provided with the female thread 915. Accordingly, after enteral nutrition has been carried out, liquid matter (an enteral nutrient) tends to remain in a gap 916 between the male member 911 and the outer cylinder 913 (e.g., in the valley of the female thread 915).

When the male connector 910 is arranged at the upstream end of the catheter inserted into a patient, the male connector 910 is indwelled together with the catheter in the patient for a long period of time. If the male connector 910 continues to be indwelled in the patient with residual liquid matter, the male connector 910 may become unsanitary. Furthermore, solidified substances formed by liquid matter dried and solidified on the male connector 910 prevent the male connector 910 and the female connector 920 from being reconnected to each other. Accordingly, it is desirable to make the male connector 910 clean through cleaning.

Patent Document 1 describes a wiping tip for cleaning the male connector 910. The wiping tip includes a water-absorbing cylindrical portion. The cylindrical portion is inserted into the gap 916 between the male member 911 and the outer cylinder 913. Liquid matter remaining in the gap is absorbed by the cylindrical portion and wiped off.

Patent Document 2 discloses a cotton swab including a cylindrical member and a cotton-like wiping member used to clean precision instruments or to remove earwax.

### Citation List

### Patent Document

Patent Document 1: JP 2017-099738A
Patent Document 2: JP 2009 100893 A

### Disclosure of Invention

### Problem to be Solved by the Invention

Patent Document 1 describes that the cylindrical portion is constituted by a foam or a non-woven fabric made of urethane or polyvinyl alcohol, and can be produced through press molding, transfer molding, or the like. However, it is difficult to mold a foam or a non-woven fabric made of the above-mentioned materials into a cylindrical portion with desired strength. If the cylindrical portion is too soft, the cylindrical portion is likely to deform when it is inserted into the gap 916 between the male member 911 and the outer cylinder 913. Accordingly, the cylindrical portion cannot be inserted deep enough to reach the bottom plate 914, and it is difficult to wipe off liquid matter adhering to the vicinity of the bottom plate 914. On the other hand, if the cylindrical portion is too hard, the cylindrical portion cannot deform to match the male connector 910. Accordingly, for example, it is difficult to wipe off liquid matter adhering to the inside of the groove of the female thread 915 or a corner in the vicinity of the bottom plate 914. In this manner, conventional wiping tips are problematic in that it is not possible to completely wipe off liquid matter adhering to the male connector 910.

It is an object of the present invention to provide a wiping tip capable of cleanly cleaning a male connector provided with a female thread formed so as to surround a male member.

### Means for Solving Problem

The present invention is directed to a wiping tip for use to wipe clean a male connector, the male connector including: a cylindrical male member; an outer cylinder surrounding the male member; and a female thread formed on an inner circumferential face of the outer cylinder facing the male member, the wiping tip including: a hollow cylindrical member; and a cotton-like wiping member arranged on the cylindrical member. The cotton-like wiping member constitutes a thin film portion covering an opening at an end of the cylindrical member, and an outer circumferential portion extending from the thin film portion and covering an outer circumferential face of the cylindrical member over a predetermined length from the end; wherein the thin film portion is penetrable by the male member of the male connector such that, in use, the male member enters the cylindrical member via the opening of the cylindrical member, and the cylindrical member is inserted into a gap between the male member and the outer cylinder.

### Effects of the Invention

The cylindrical member makes it possible to deeply insert the cotton-like wiping member into a gap between a male member and an outer cylinder. The cotton-like wiping member is interposed between a cylindrical member and a male connector. Thus, according to the present invention, it is possible to cleanly clean a male connector provided with a female thread formed so as to surround a male member.

### Brief Description of Drawings

[FIG. 1] FIG. 1A is a perspective view of a wiping tip according to Embodiment 1 of the present invention. FIG. 1B is a cross-sectional view of the wiping tip according to Embodiment 1 of the present invention.
[FIG. 2] FIG. 2 is a cross-sectional view showing a step of cleaning the male connector using the wiping tip according to Embodiment 1 of the present invention.
[FIG. 3] FIG. 3 is a cross-sectional view showing a next step of cleaning the male connector using the wiping tip according to Embodiment 1 of the present invention.
[FIG. 4] FIG. 4 is a cross-sectional view showing a next step of cleaning the male connector using the wiping tip according to Embodiment 1 of the present invention.
[FIG. 5] FIG. 5 is a cross-sectional view showing a next step of cleaning the male connector using the wiping tip according to Embodiment 1 of the present invention.
[FIG. 6] FIG. 6A is a perspective view of another wiping tip according to Embodiment 1 of the present invention. FIG. 6B is a cross-sectional view of the wiping tip in FIG. 6A.
[FIG. 7] FIG. 7A is an enlarged perspective view of part of a wiping tip according to Embodiment 2 of the present invention. FIG. 7B is an enlarged cross-sectional view of part of the wiping tip according to Embodiment 2 of the present invention.
[FIG. 8] FIG. 8 is a cross-sectional view of a wiping tip according to Embodiment 3 of the present invention.
[FIG. 9] FIG. 9 is a cross-sectional view of a wiping tip according to Embodiment 4 of the present invention.
[FIG. 10] FIG. 10 is a cross-sectional view of a wiping tip according to Embodiment 5 of the present invention.
[FIG. 11] FIG. 11A is a perspective view of a male connector that is used as a patient-side connector in enteral nutrition. FIG. 11B is a cross-sectional view taken along a face including a central axis of the male connector.
[FIG. 12] FIG. 12A is a perspective view of a female connector that is used as a container-side connector in enteral nutrition. FIG. 12B is a cross-sectional view taken along a face including a central axis of the female connector.

### Description of the Invention

In the wiping tip of the present invention, the cotton-like wiping member may be arranged at both ends of the cylindrical member. According to this aspect, for example, a male connector can be cleanly cleaned by sequentially using the cotton-like wiping members at the two ends.

Alternatively, the cotton-like wiping member may be arranged at only one end of the cylindrical member. This aspect is advantageous in urging a user to dispose of the wiping tip of the present invention after single use.

The cotton-like wiping member may be impregnated with a cleaning solution. This aspect is advantageous in cleanly cleaning the male connector.

A slit may be formed through the thin film portion. This aspect is advantageous, first, in allowing the male member to pierce the thin film portion with ease, and, second, in uniformly wiping clean the outer circumferential face of the male member.

A cotton-like filling member may be placed in the cylindrical member. This aspect is advantageous in wiping off liquid matter adhering to the end of the male member.

In the wiping tip of the present invention, the wiping tip may include a stick-shaped rod. Furthermore, a hole may be provided over a predetermined length extending along a longitudinal direction of the rod, from an end face of the rod. The portion of the rod in which the hole is provided may be the cylindrical member. The portion of the rod other than the cylindrical member is solid, and has relatively high strength. The wiping tip including this rod is advantageous in efficiently performing cleaning.

In the wiping tip of the present invention, one end thereof may include the cylindrical member provided with the cotton-like wiping member, and another end thereof may include a handle. This aspect is advantageous in improving the cleaning efficiency and keeping the cotton-like wiping member clean.

In the wiping tip of the present invention, the wiping tip may be used to wipe clean a male connector including: a cylindrical male member; an outer cylinder surrounding the male member; and a female thread formed on an inner circumferential face of the outer cylinder facing the male member. According to this aspect, a male connector that is used as a patient-side connector in enteral nutrition can be cleaned using the wiping tip of the present invention.

The thin film portion is penetrable by the male member, and the cylindrical member is insertable into a gap between the male member and the outer cylinder. According to this aspect, the end of the male member and the faces of the male member and the outer cylinder defining the gap can be wiped clean.

In the wiping tip of the present invention, the thin film portion may be configured to be penetrated by the male member and folded into the cylindrical member. According to this aspect, the folded thin film portion wipes off liquid matter adhering to the outer circumferential face of the male member.

An outer diameter of the outer circumferential portion may be larger than an inner diameter of the female thread. This aspect is advantageous in wiping off liquid matter adhering to the inside of the groove of the female thread.

An outer diameter of the cylindrical member may be equal to or smaller than an inner diameter of the female thread. According to this aspect, the wiping tip can be deeply inserted into the gap between the male member and the outer cylinder. This aspect is advantageous in cleanly cleaning the male connector.

An inner diameter of the cylindrical member may be equal to or larger than a maximum outer diameter of an outer circumferential face of the male member. This aspect is advantageous in the following points. First, the wiping tip can be deeply inserted into the gap between the male member and the outer cylinder. It is advantageous in cleanly cleaning the male connector. Second, the thin film portion torn by the male member can be reliably interposed between the male member and the cylindrical member. It is advantageous in preventing the cylindrical member from damaging the outer circumferential face of the male member.

Hereinafter, the present invention will be described in detail while showing preferred embodiments thereof. However, it will be appreciated that the present invention is not limited to the embodiments below. In the drawings that will be referred to in the following description, only the main members constituting the embodiments of the present invention are shown in a simplified manner for the sake of convenience of description. Therefore, an optional member that is not shown in the drawings referred to below may be included. Furthermore, any of the members shown in the drawings may be changed or omitted, within the scope of the present invention. In the drawings that will be referred to in the description of the embodiments below, members corresponding to those shown in the drawings that are referred to in the description of any preceding embodiment are denoted by the same reference numerals as those of the members shown in the drawings of that preceding embodiment. With respect to such members, redundant descriptions are omitted, and the description of the preceding embodiment should be taken into account.

### Embodiment 1

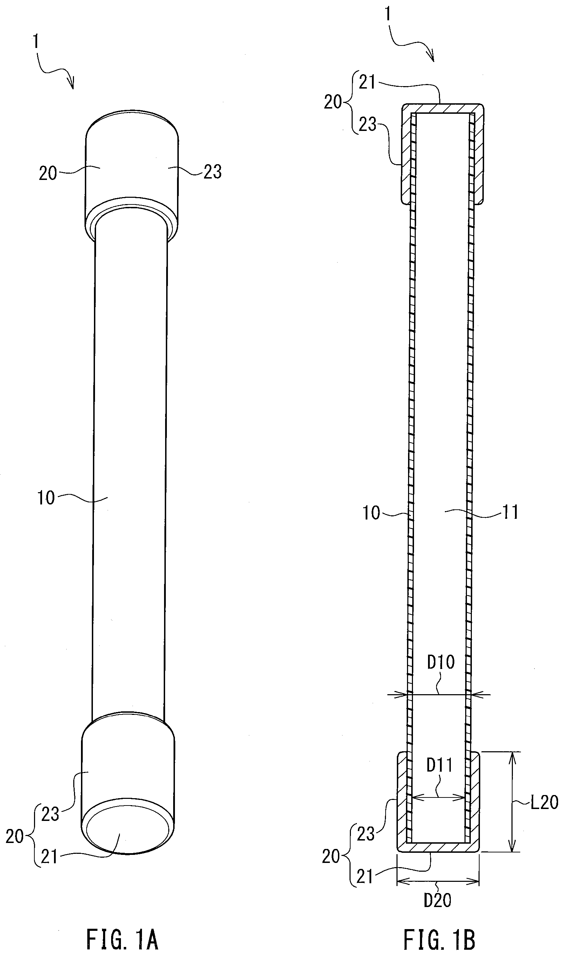

FIG. 1A is a perspective view of a wiping tip 1 according to Embodiment 1 of the present invention. FIG. 1B is a cross-sectional view of the wiping tip 1. The wiping tip 1 includes an elongated stick-shaped cylindrical member (rod) 10 extending straight, and a cotton-like wiping member 20 arranged at both ends of the cylindrical member 10.

The cylindrical member 10 has a hollow cylindrical shape in which a hole 11 extending along the longitudinal direction of the cylindrical member 10 is formed through the cylindrical member 10. The hole 11 is a through-hole connecting the two ends of the cylindrical member 10, and is connected to the outside via openings at the two ends of the cylindrical member 10. The outer circumferential face and the inner circumferential face of the cylindrical member 10 both have a circular cross-sectional shape. The inner diameter and the outer diameter of the cylindrical member 10 are each constant along the longitudinal direction of the cylindrical member 10. The cylindrical member 10 is strong enough so as to not allow the cylindrical member to substantially deform when it is pinched by user's fingers or when a bending force or a torsional force is applied thereto. There is no limitation on the material of the cylindrical member 10, but examples thereof include resin materials such as polyethylene, polypropylene, polycarbonate, styrene ethylene, polyethylene terephthalate, polybutylene terephthalate, a butylene styrene block copolymer, polyacetal, polyamide, and hard polyvinyl chloride, and, of these materials, it is preferable to use polyolefin-based resins such as polyethylene and polypropylene. It is preferable that the cylindrical member 10 is integrally produced as a single component through injection molding of the above-described resin materials.

The cotton-like wiping member 20 is a cotton-like material in which fibers are entwined. There is no limitation on the material of the cotton-like wiping member 20, but examples thereof include water-absorbing materials such as cotton and rayon, and it is particularly preferable to use absorbent cotton. The cotton-like wiping member 20 constitutes a thin film portion 21 in the shape of a thin film covering an opening at an end of the cylindrical member 10, and an outer circumferential portion 23 extending from the thin film portion 21. The outer circumferential portion 23 covers the outer circumferential face of the cylindrical member 10, over a predetermined length from the end of the cylindrical member 10. The cotton-like wiping member 20 is arranged at an end of the cylindrical member 10 such that the thin film portion 21 and the outer circumferential portion 23 are continuously formed.

There is no limitation on the method for attaching the cotton-like wiping member 20 to the cylindrical member 10, and, for example, it is possible to use substantially the same method as a method for producing a cotton swab. That is to say, a cotton-like material with a predetermined size is pressed against the end of the cylindrical member 10 so as to close the opening of the cylindrical member 10, and the cylindrical member 10 is rotated so that the cotton-like material is wound around the outer circumferential face of the cylindrical member 10. It is also possible to firmly fix the cotton-like wiping member 20 to the cylindrical member 10 using an adhesive made of PVA (polyvinyl alcohol) or the like. The adhesive is preferably applied to only part (e.g., the portion of the outer circumferential portion 23 farthest from the end of the cylindrical member 10) of the outer circumferential portion 23. The cotton-like wiping member 20 is soft and elastic except for the portion to which the adhesive is applied.

Hereinafter, a method for using the wiping tip 1 will be described.

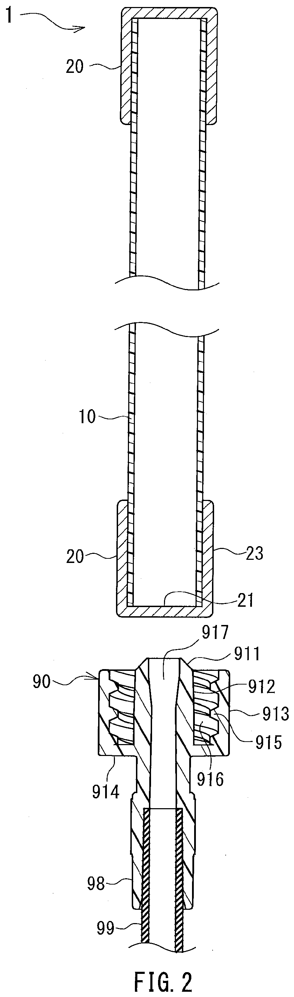

The wiping tip 1 can be used to clean a male connector 90 shown in FIG. 2. As in the case of the male connector 910 in FIGS. 11A and 11B, the male connector 90 is a patient-side connector in enteral nutrition. Among the elements constituting the male connector 90, the same elements as those constituting the male connector 910 in FIGS. 11A and 11B are denoted by the same reference numeral as those in FIGS. 11A and 11B, and a description thereof has been omitted. The male connector 90 includes a hollow base end 98 connected to the flow path 917 of the male member 911. A flexible tube 99 is inserted into and connected to the base end 98. The tube 99 may be a catheter (a nasal catheter, a PEG catheter, etc.) indwelled in a patient. Alternatively, the tube 99 may be a tube connected to the catheter. The catheter is inserted into the gastrointestinal tract (e.g., the stomach) of a patient.

When carrying out enteral nutrition, a female connector (see FIGS. 12A and 12B) is connected to the male connector 90, and liquid matter (e.g., an enteral nutrient) is administered via these connectors to the patient. Subsequently, the female connector is separated from the male connector 90. As described above, liquid matter (not shown) has adhered to the separated male connector 90. The male connector 90 in FIG. 2 is in this state.

First, as shown in FIG. 2, the thin film portion 21 at an end of the wiping tip 1 is positioned to face the male connector 90. An operator can grip the exposed outer circumferential face of the cylindrical member 10.

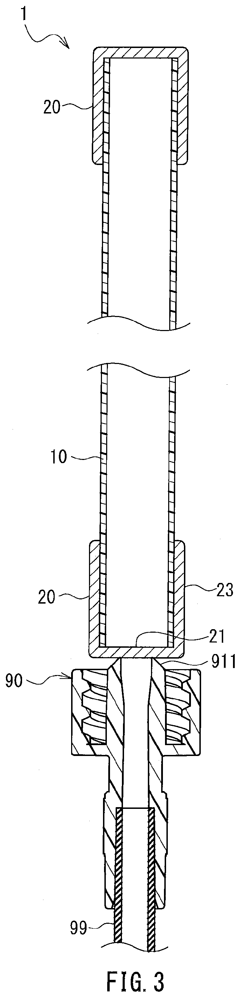

Next, as shown in FIG. 3, the thin film portion 21 at the end of the wiping tip 1 is brought into contact with the end of the male member 911 of the male connector 90. The wiping tip 1 is aligned substantially coaxially with the male member 911. Then, the wiping tip 1 is pressed against the male connector 90.

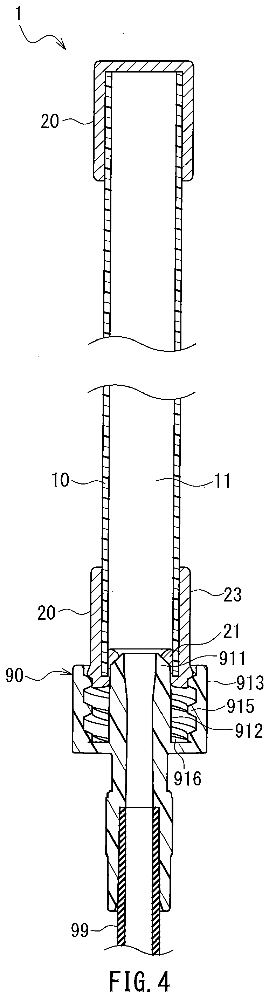

As shown in FIG. 4, the cylindrical member 10 is inserted into a gap 916 between the male member 911 and the outer cylinder 913. The thin film portion 21 is pressed against the end of the male member 911. Since the thin film portion 21 is a thin film constituted by the cotton-like wiping member 20, it is relatively easily pierced by the male member 911. The male member 911 penetrates the thin film portion 21 and enters the hole 11 via the opening of the cylindrical member 10. The torn thin film portion 21 is folded into the hole 11 of the cylindrical member 10 so as to be held between the male member 911 and the cylindrical member 10.

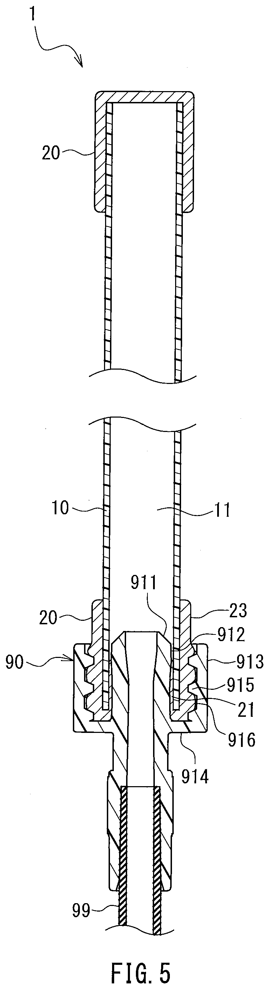

The wiping tip 1 is rotated in a clockwise direction with respect to the male connector 90 when viewed from the wiping tip 1 side. The outer circumferential portion 23 of the cotton-like wiping member 20 moves along the female thread 915 of the male connector 90. Even when a rotational force is applied to the wiping tip 1, the cylindrical member 10 does not substantially deform. In accordance with the rotation of the wiping tip 1, the wiping tip 1 enters (moves forward) deeper into the gap 916 of the male connector 90. Finally, as shown in FIG. 5, the end of the wiping tip 1 comes into contact with a bottom plate 914 of the male connector 90. An operator can recognize that the end of the wiping tip 1 has come into contact with the bottom plate 914 when the rotational force necessary to rotate the wiping tip 1 increases.

Subsequently, the wiping tip 1 is rotated in the opposite direction (the counterclockwise direction) with respect to the male connector 90. The outer circumferential portion 23 of the cotton-like wiping member 20 moves along the female thread 915 of the male connector 90. In accordance with the rotation of the wiping tip 1, the cylindrical member 10 gradually moves backward from the gap 916 of the male connector 90. Then, the wiping tip 1 is separated from the male connector 90. The used wiping tip 1 is then disposed of.

When the wiping tip 1 is inserted into the male connector 90 and then pulled out from the male connector 90 as described above, the male connector 90 can be cleaned by allowing the cotton-like wiping member 20 to wipe off liquid matter adhering to the male connector. The liquid matter is removed from the male connector 90 in a state of being absorbed by the cotton-like wiping member 20 and retained in the cotton-like wiping member 20. Solid substances contained in the liquid matter are also wiped off and removed by the cotton-like wiping member 20 together with the liquid matter.

In the above-described conventional wiping tips, the entire cylindrical portion configured to be inserted into the gap 916 of the male connector is made of a water-absorbing material. Accordingly, it is difficult to produce a cylindrical portion with desired strength. If the cylindrical portion is too soft or too hard, it is not possible to completely wipe off liquid matter.

On the other hand, in the wiping tip 1 of Embodiment 1, the hollow cylindrical member 10 is provided with the cotton-like wiping member 20.

The cylindrical member 10 functioning as a core can be made of a material with desired strength. Accordingly, even when the wiping tip 1 is moved forward and backward while it is rotated with respect to the male connector 90, the cylindrical member 10 does not substantially deform. It is easy to insert the cotton-like wiping member 20 arranged on the cylindrical member 10 into the gap 916 until the cotton-like wiping member 20 comes into contact with the bottom plate 914.

The cotton-like wiping member 20 is likely to deform to match the shape of the surface of the male connector 90 with which the cotton-like wiping member 20 has been brought into contact. The cotton-like wiping member 20 continuously extends from the thin film portion 21 covering an opening of the cylindrical member 10 to the outer circumferential portion 23 covering the outer circumferential face of the cylindrical member 10. During the process in which the wiping tip 1 is moved forward and backward while it is rotated with respect to the male connector 90, the outer circumferential portion 23 of the cotton-like wiping member 20 wipes clean the female thread 915 formed on the inner circumferential face of the outer cylinder 913. The outer circumferential portion 23 deforms to match the thread shape of the female thread 915, enters the groove of the female thread 915, and wipes off liquid matter adhering to the groove. The cotton-like wiping member 20 covering the circular end of the cylindrical member 10 wipes off liquid matter adhering to the bottom plate 914, a corner at the boundary between the bottom plate 914 and the outer cylinder 913, and a corner at the boundary between the bottom plate 914 and the male member 911.

In an initial state, the thin film portion 21 of the cotton-like wiping member 20 covers an opening of the cylindrical member 10. During the process in which the male member 911 penetrates the thin film portion 21 (see FIG. 4), the thin film portion 21 wipes off liquid matter adhering to the end of the male member 911. In the case of conventional wiping tips, in order to clean the end of the male member 911, an end of a water-absorbing cylindrical portion needs to be intentionally brought into contact with the end of the male member 911 before the cylindrical portion is inserted into the gap 916. Without this process, it is not possible to wipe off liquid matter at the end of the male member 911. On the other hand, in the case of Embodiment 1, it is possible to allow the thin film portion 21 to wipe clean the end of the male member 911, merely by pushing the wiping tip 1 into the male connector 90.

The thin film portion 21 penetrated by the male member 911 is folded into the hole 11 of the cylindrical member 10 and is held between the male member 911 and the cylindrical member 10. When the wiping tip 1 is pushed into and pulled out from the male connector 90, the thin film portion 21 moves along the outer circumferential face 912 of the male member 911, and wipes off liquid matter adhering to the outer circumferential face 912.

In this manner, according to Embodiment 1, the strength necessary for the wiping tip 1 is ensured by the cylindrical member 10. During the cleaning of the male connector 90 using the wiping tip 1, the cotton-like wiping member 20 is always interposed between the cylindrical member 10 and the male connector 90. When the wiping tip 1 is pushed into and pulled out from the male connector 90, the cotton-like wiping member 20 moves while remaining in contact with the male connector 90. Accordingly, it is possible to completely wipe off liquid matter adhering to the male connector 90. The wiping tip 1 is advantageous in cleanly cleaning the male connector 90.

In a used wiping tip 1, the thin film portion 21 has been torn. It is possible to easily determine whether or not the wiping tip has been used, by looking at the thin film portion 21. The possibility of using a used wiping tip 1 again by mistake is low. According to this aspect as well, the wiping tip 1 is advantageous in cleanly cleaning the male connector 90.

The cylindrical member 10 has a simple hollow cylindrical shape. The cotton-like wiping member 20 can be wound around the end of the cylindrical member 10 using substantially the same method as a method for producing a cotton swab. Accordingly, the wiping tip 1 has a simple structure and can be easily produced. The wiping tip 1 can be produced in large quantities at low cost.

An outer diameter D₂₀ (see FIG. 1B) of the outer circumferential portion 23 of the wiping tip 1 before cleaning of the male connector 90 (in an unused state) is preferably larger than the inner diameter of the female thread 915 of the male connector 90. The reason for this is that the outer circumferential portion 23 can be allowed to enter the groove of the female thread 915. If the outer diameter D₂₀ of the outer circumferential portion 23 is equal to or smaller than the inner diameter of the female thread 915, the ability to wipe off liquid matter in the groove of the female thread 915 is diminished. Specifically, the outer diameter D₂₀ preferably satisfies D₂₀ ≥ 8.55 mm. The upper limit of the outer diameter D₂₀ of the outer circumferential portion 23 is not limited to this, but D₂₀ ≤ 12 mm is preferably satisfied. If the outer diameter D₂₀ is too large, it is difficult to insert the wiping tip 1 into the gap 916 until the end of the wiping tip 1 comes into contact with the bottom plate 914, and thus, in particular, the ability to wipe off liquid matter adhering to the vicinity of the bottom plate 914 is diminished.

A length L₂₀ (see FIG. 1B) of the outer circumferential portion 23 along the longitudinal direction of the cylindrical member 10 is preferably equal to or greater than the depth of the gap 916 of the male connector 90 (the distance from the bottom plate 914 to the end of the outer cylinder 913). If the length L₂₀ of the outer circumferential portion 23 is less than this depth, the ability to wipe off liquid matter adhering to the inside of the groove of the female thread 915 and the bottom plate 914 is diminished. Specifically, the length L₂₀ preferably satisfies L₂₀ ≥ 6.82 mm.

An outer diameter D₁₀ (see FIG. 1B) of the cylindrical member 10 is preferably equal to or smaller than the inner diameter of the female thread 915 of the male connector 90, and more preferably smaller than the inner diameter of the female thread 915. If the outer diameter D₁₀ of the cylindrical member 10 is larger than the inner diameter of the female thread 915, the wiping tip 1 cannot be inserted into the gap 916 of the male connector 90, and thus the male connector 90 cannot be cleaned with the wiping tip 1. Note that, if the outer diameter D₁₀ of the cylindrical member 10 is excessively smaller than the inner diameter of the female thread 915, it is difficult to allow the cotton-like wiping member 20 constituting the outer circumferential portion 23 to enter the groove of the female thread 915, and thus the ability to wipe off liquid matter in the groove of the female thread 915 is diminished.

An inner diameter D₁₁ (see FIG. 1B) of the cylindrical member 10 is preferably equal to or larger than the maximum outer diameter of the outer circumferential face 912 of the male member 911 of the male connector 90, and more preferably larger than the maximum outer diameter of the outer circumferential face 912. If the inner diameter D₁₁ of the cylindrical member 10 is smaller than the maximum outer diameter of the outer circumferential face 912, the following two issues may arise. First, it is difficult to insert the wiping tip 1 into the gap 916 until the end of the wiping tip comes into contact with the bottom plate 914. Accordingly, in particular, the ability to wipe off liquid matter adhering to the vicinity of the bottom plate 914 is diminished. Second, the thin film portion 21 (see FIG. 5) held between the male member 911 and the cylindrical member 10 becomes thinner, as a result of which the cylindrical member 10 comes into direct contact with the male member 911. Accordingly, the outer circumferential face 912 of the male member 911 may be damaged by the cylindrical member 10. Specifically, the inner diameter D₁₁ preferably satisfies the inner diameter D₁₁ ≥ 5.59 mm. Note that, if the inner diameter D₁₁ of the cylindrical member 10 is excessively larger than the maximum outer diameter of the outer circumferential face 912, the ability of the torn thin film portion 21 to wipe off liquid matter adhering to the outer circumferential face 912 is diminished. Accordingly, the difference between the inner diameter D₁₁ of the cylindrical member 10 and the maximum outer diameter of the outer circumferential face 912 (the inner diameter of the cylindrical member 10 - the maximum outer diameter of the outer circumferential face 912) is preferably equal to or less than twice the thickness of an unused thin film portion 21, and more preferably equal to or less than the thickness of an unused thin film portion 21.

The cotton-like wiping member 20 may be impregnated with a cleaning solution. There is no limitation on the cleaning solution, but examples thereof include alcohol and sterilized purified water. Solidified substances and the like formed by liquid matter dried and stuck to the male connector 90 can be removed clean using the cleaning solution.

In the wiping tip 1, the cotton-like wiping member 20 is arranged at both ends of the cylindrical member 10. Accordingly, first, most of the liquid matter adhering to the male connector 90 can be wiped off using one of the cotton-like wiping members 20, and then a slight amount of still remaining liquid matter can be wiped off clean using the other cotton-like wiping member 20. It is possible to cleanly clean the male connector 90 with a small number of wiping tips 1.

The two ends of the wiping tip 1 may be symmetrical or asymmetrical. For example, one of the cotton-like wiping members 20 may be formed thicker than the other cotton-like wiping member 20. This aspect is advantageous, for example, in wiping clean a plurality of types of male connectors 90 with different dimensions, using a common wiping tip.

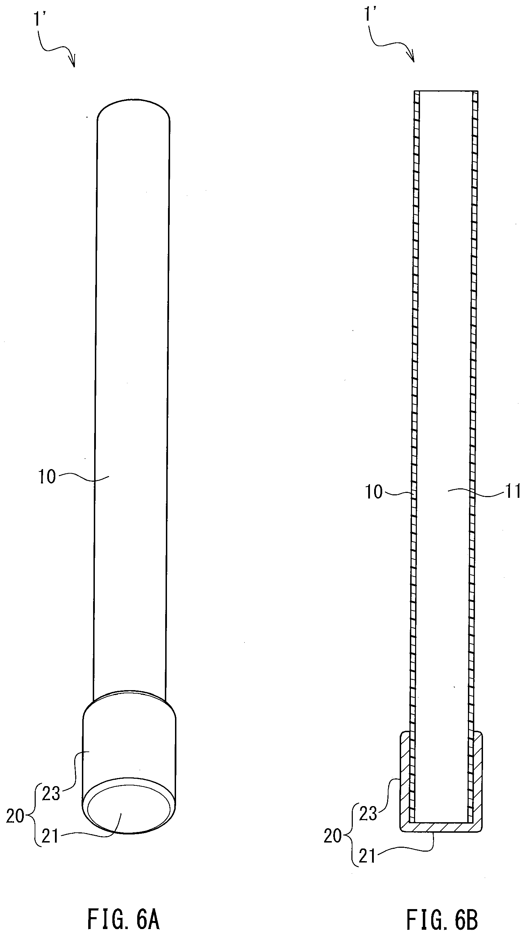

In the wiping tip of the present invention, the cotton-like wiping member 20 does not have to be arranged at both ends of the cylindrical member (rod) 10, and, as in a wiping tip 1' in FIGS. 6A and 6B, the cotton-like wiping member 20 may be arranged at only one end of the cylindrical member 10. In the case in which the cotton-like wiping member 20 is arranged at both ends of the cylindrical member 10 as in the wiping tip 1 in FIGS. 1A and 1B, a user may use only one of the cotton-like wiping members (first cotton-like wiping member) 20 in cleaning of the male connector 90, and store the wiping tip 1 without disposing of it until next cleaning of the male connector 90 in order to use the other cotton-like wiping member (second cotton-like wiping member) 20 in the next cleaning. In this case, the hygienic state of the second cotton-like wiping member 20 may deteriorate depending on the storage condition. With the wiping tip 1' in which the cotton-like wiping member 20 is arranged at only one end, the user can be urged to dispose of the wiping tip 1' after single use, which is advantageous in ensuring cleaning of the male connector 90 always using a clean cotton-like wiping member 20.

### Embodiment 2

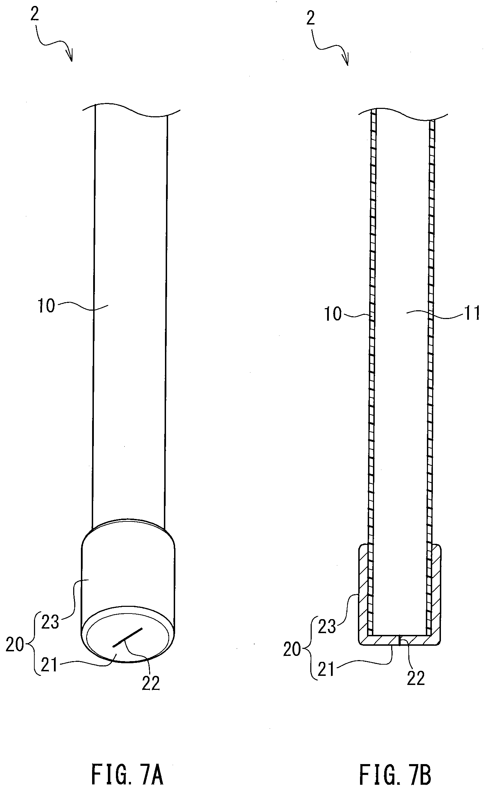

FIG. 7A is an enlarged perspective view of part of a wiping tip 2 according to Embodiment 2 of the present invention, and FIG. 7B is an enlarged cross-sectional view of part of the wiping tip 2. In Embodiment 2, a slit 22 is formed through the thin film portion 21. The slit 22 is a linear notch in the shape of a "-" (minus) in a plan view, and passes through the center of the circular thin film portion 21. The slit 22 is formed through the thin film portion 21. It is easy to form the slit 22 with a sharp knife or the like after the cotton-like wiping member 20 is arranged at an end of the cylindrical member 10 in a similar way to that of Embodiment 1.

The wiping tip 2 of Embodiment 2 can be used to clean the male connector 90 as in the case of the wiping tip 1 of Embodiment 1.

In Embodiment 2, when the end of the male member 911 of the male connector 90 is pressed against the thin film portion 21, the thin film portion 21 is torn from the slit 22 (particularly two ends thereof). This aspect has the following effects. First, the thin film portion 21 is easily torn. Accordingly, the male member 911 can penetrate the thin film portion 21 merely with application of a relatively small force. Even an inexperienced operator can easily insert the wiping tip 2 into the gap 916 of the male connector 90. Second, the thin film portion 21 is torn always in a stable manner, for example, such as the thin film portion 21 being torn into substantially two equal parts. Even when the male member 911 is pressed against a position slightly deviated from the center of the thin film portion 21, there is little variation in the torn manner. When the male member 911 is inserted into the cylindrical member 10, the torn thin film portion 21 surrounds the outer circumferential face 912 of the male member 911 substantially evenly in the circumferential direction. This aspect is advantageous in uniformly wiping off liquid matter adhering to the outer circumferential face 912.

The slit 22 may be formed through both thin film portions 21 at the respective ends of the wiping tip 2, or only one of the thin film portions 21. The shape of the slit 22 is not limited to that in this embodiment, and may be changed as necessary.

Embodiment 2 is the same as Embodiment 1, except for the above-described aspects. The description of Embodiment 1 applies also to Embodiment 2.

### Embodiment 3

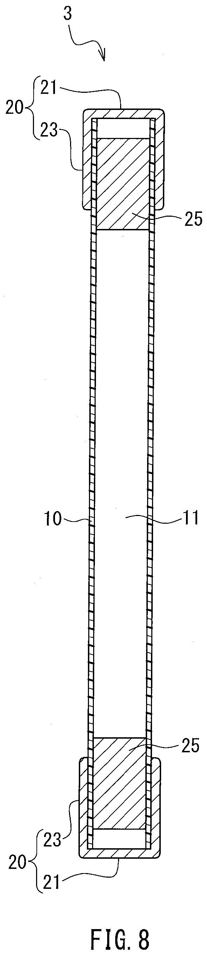

FIG. 8 is a cross-sectional view of a wiping tip 3 according to Embodiment 3 of the present invention. In Embodiment 3, cotton-like filling members 25 are placed in the hole 11 of the cylindrical member 10. Each cotton-like filling member 25 is a cotton-like material in which fibers are entwined, and there is no limitation on the material, but examples thereof include the same materials as those of the cotton-like wiping member 20. The cotton-like filling member 25 is arranged in the vicinity of an opening of the cylindrical member 10. The distance from the end of the cylindrical member 10 to the cotton-like filling member 25 is less than the length of the male member 911 of the male connector 90 (the protruding length of the male member 911 from the bottom plate 914). The cotton-like filling member 25 may be in contact with the thin film portion 21.

The wiping tip 3 of Embodiment 3 can be used to clean the male connector 90 as in the case of the wiping tip 1 of Embodiment 1.

In Embodiment 3, when the male member 911 of the male connector 90 penetrates the thin film portion 21 and enters the hole 11, the cotton-like filling member 25 comes into contact with the end of the male member 911. When the wiping tip 3 is rotated and moved forward to the male connector 90, the male member 911 gradually compresses or moves the cotton-like filling member 25 while rotating with respect to the cotton-like filling member 25. Even in the case in which liquid matter adhering to the end of the male member 911 is not completely wiped off by the thin film portion 21 when the male member 911 pierces the thin film portion 21, the liquid matter can be wiped off later by the cotton-like filling member 25.

Not only portions in the vicinity of the two ends of the hole 11 of the cylindrical member 10 but also a portion over substantially the entire length of the hole 11 may be filled with the cotton-like filling member 25.

The cotton-like filling member 25 may be impregnated with the cleaning solution described in Embodiment 1.

Embodiment 3 is the same as Embodiment 1, except for the above-described aspects. The description of Embodiment 1 applies also to Embodiment 3. Embodiment 3 may be applied to the wiping tip 2 of Embodiment 2.

### Embodiment 4

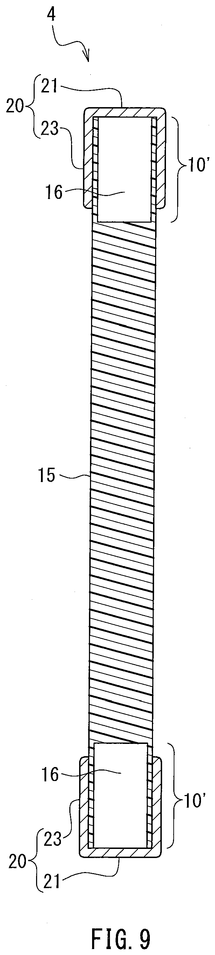

FIG. 9 is a cross-sectional view of a wiping tip 4 according to Embodiment 4 of the present invention. In Embodiments 1 to 3, the entirety of the stick-shaped rod corresponding to a core of the wiping tips 1 to 3 is constituted by the hollow cylindrical member 10. On the other hand, in the wiping tip 4 of Embodiment 4, only part (the ends) of the stick-shaped rod is constituted by the hollow cylindrical member. That is to say, holes 16 extend from the two end faces of an elongated stick-shaped rod 15 extending straight, along the longitudinal direction of the rod 15. The holes 16 are non-through-holes that are not formed extending through the rod 15. In a similar way to that of Embodiment 1, the cotton-like wiping members 20 are arranged so as to cover the openings of the holes 16. In Embodiment 4, portions 10' of the rod 15 in which the holes 16 are provided correspond to a "cylindrical member" of the present invention. The portion of the rod 15 other than the cylindrical members 10' at the two ends is a solid columnar member. There is no limitation on the rod 15, but it is preferable that the rod 15 is integrally produced as a single component through injection molding of the resin materials described as examples of the material of the cylindrical member 10 in Embodiment 1.

The wiping tip 4 of Embodiment 4 can be used to clean the male connector 90 as in the case of the wiping tip 1 of Embodiment 1. The male member 911 pierces the thin film portion 21 and is inserted into the holes 16. The depth of each of the holes 16 is more than the length of the male member 911 of the male connector 90 (the protruding length of the male member 911 from the bottom plate 914).

Since the portion of the rod 15 other than the portions in which the holes 16 are formed is a solid columnar member, it has relatively high strength. Accordingly, the rod 15 is not crushed in the diameter direction or bent by a force applied during wipe cleaning. It is possible to efficiently perform cleaning.

The hole 16 and the cotton-like wiping member 20 may be arranged at only one end of the rod 15.

Embodiment 4 is the same as Embodiment 1, except for the above-described aspects. The description of Embodiment 1 applies also to Embodiment 4. Embodiment 4 may be applied to the wiping tips 2 and 3 of Embodiments 2 and 3.

### Embodiment 5

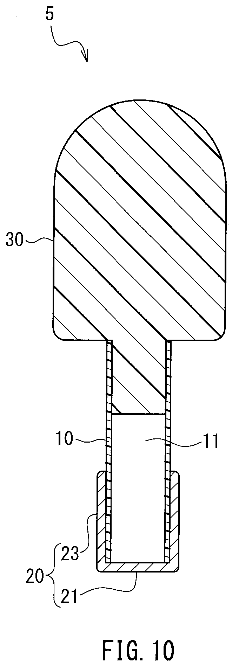

FIG. 10 is a cross-sectional view of a wiping tip 5 according to Embodiment 5 of the present invention. In Embodiment 5, the cylindrical member 10 is relatively short. One end of the cylindrical member 10 is provided with the cotton-like wiping member 20, and the other end (base end) of the cylindrical member 10 is provided with a handle 30. The handle 30 is a block object with an outer diameter larger than that of the cylindrical member 10. It is preferable that the handle 30 is strong enough so as to not allow the handle 30 to deform with ease when a force is applied thereto by an operator. There is no limitation on the material of the handle 30, but examples thereof include resin materials such as polyethylene, polypropylene, polycarbonate, styrene ethylene, polyethylene terephthalate, polybutylene terephthalate, a butylene styrene block copolymer, polyacetal, polyamide, and hard polyvinyl chloride.

The wiping tip 5 of Embodiment 5 can be used to clean the male connector 90 as in the case of the wiping tip 1 of Embodiment 1.

An operator can perform wipe cleaning while gripping the handle 30. The handle 30 is relatively large and is unlikely to deform, and thus the operator can firmly grip the handle 30. It is also easy to apply a rotational force to the wiping tip 5. Accordingly, the cleaning efficiency is improved. Furthermore, since the operator is unlikely to touch the cotton-like wiping member 20, the cotton-like wiping member 20 is kept clean.

The cylindrical member 10 may be integrated with the handle 30 so as not to be separated therefrom. There is no limitation on the method for integrating the cylindrical member 10 and the handle 30, but examples thereof include a method in which the cylindrical member 10 and the handle 30 are separately produced, after which they are connected to each other into one piece using an adhesive, welding, or the like, and a method in which one of the cylindrical member 10 and the handle 30 is produced, after which the other is integrated with the one through double-molding. Alternatively, it is also possible to integrally mold the cylindrical member 10 and the handle 30 simultaneously as one component using the same material.

The cylindrical member 10 may be detachably attached to the handle 30. In this case, the handle 30 can be repeatedly used while the cylindrical member 10 provided with the cotton-like wiping member 20 is disposed of after single use. In order to allow a rotational force applied to the handle 30 to be reliably transferred to the cylindrical member 10, the handle 30 and the cylindrical member 10 may have a fitting shape (e.g., a projection and a recess) in which they are fitted to each other at the corresponding connecting portions.

In FIG. 10, part of the handle 30 is fitted into the hole 11 of the cylindrical member 10, but the structure for connecting the cylindrical member 10 and the handle 30 is not limited to this. For example, the cylindrical member 10 may be fitted into a hole formed in the handle 30.

In FIG. 10, the cylindrical member 10 is connected to the handle 30, but the present invention is not limited to this. As in the case of the rod 15 of Embodiment 4, a solid columnar member integrally produced with the cylindrical member may be connected to the handle.

Embodiment 5 is the same as Embodiment 1, except for the above-described aspects. The description of Embodiment 1 applies also to Embodiment 5. Embodiment 5 may be applied to the wiping tips 2 to 4 of Embodiment 2 to 4.

Embodiments 1 to 5 above are merely an example. The present invention is not limited to Embodiments 1 to 5, and may be changed as appropriate.

In the foregoing embodiments, the outer circumferential face and the inner circumferential face of the cylindrical member both have a circular cross-sectional shape, but the present invention is not limited to this. For example, one or both of the outer circumferential face and the inner circumferential face may have a cross-section in the shape of a regular polygon (e.g., a regular octagon, etc.). The outer circumferential face having such a cross-sectional shape is advantageous in applying a rotational force to the wiping tip.

In the foregoing embodiments, the inner circumferential face of the cylindrical member is a cylindrical face whose inner diameter is constant in the longitudinal direction, but the present invention is not limited to this. In a portion into which the male member 911 is inserted, the inner circumferential face of the cylindrical member may have a female tapered surface whose inner diameter increases toward the opening (e.g., a female tapered surface with the same taper angle as that of the outer circumferential face 912 of the male member 911).

The wiping tip of the present invention may be of a disposable type in which the wiping tip is disposed of after each cleaning of a male connector, or of a reusable type in which the wiping tip is repeatedly used to clean a male connector through washing with water or the like and drying after cleaning of a male connector.

### Industrial Applicability

While there is no limitation on the field of use of the present invention, the present invention can be preferably used as a cleaning tool for a male connector including a male member, and an outer cylinder provided with a female thread. In particular, the present invention is suitable as a cleaning tool for a male connector arranged at an upstream end of a catheter inserted into the body of a patient in order to carry out enteral nutrition.

### List of Reference Numerals

- 1, 1', 2, 3, 4, 5: Wiping tip
- 10, 10': Cylindrical member
- 15: Rod
- 16: Hole
- 20: Cotton-like wiping member
- 21: Thin film portion
- 22: Slit
- 23: Outer circumferential portion
- 25: Cotton-like filling member
- 90: Male connector
- 911: Male member
- 912: Outer circumferential face of male member
- 913: Outer cylinder
- 915: Female thread
- 916: Gap between male member and outer cylinder

## Claims

1. A wiping tip (1; 1'; 2; 3; 4; 5) for use to wipe clean a male connector (910), the male connector including: a cylindrical male member (911); an outer cylinder (913) surrounding the male member (911); and a female thread (915) formed on an inner circumferential face of the outer cylinder (913) facing the male member (911), the wiping tip (1; 1'; 2; 3; 4; 5) comprising: a hollow cylindrical member (10); and **characterised by** a cotton-like wiping member (20) arranged on the cylindrical member (10),
wherein the cotton-like wiping member (20) constitutes a thin film portion (21) covering an opening (11) at an end of the cylindrical member (10), and an outer circumferential portion (23) extending from the thin film portion (21) and covering an outer circumferential face of the cylindrical member (10) over a predetermined length (L20) from the end;
wherein the thin film portion (21) is penetrable by the male member (911) of the male connector (910) such that, in use, the male member (911) enters the cylindrical member (10) via the opening (11) of the cylindrical member (10), and the cylindrical member (10) is inserted into a gap (916) between the male member (911) and the outer cylinder (913).

2. The wiping tip according to claim 1, wherein the cotton-like wiping member (20) is arranged at both ends of the cylindrical member (10).

3. The wiping tip according to claim 1, wherein the cotton-like wiping member (20) is arranged at only one end of the cylindrical member (10).

4. The wiping tip according to any one of claims 1 to 3, wherein the cotton-like wiping member (20) is impregnated with a cleaning solution.

5. The wiping tip according to any one of claims 1 to 4, wherein a slit (22) is formed through the thin film portion (21).

6. The wiping tip according to any one of claims 1 to 5, wherein a cotton-like filling member (25) is placed in the cylindrical member (10).

7. The wiping tip according to any one of claims 1 and 3 to 6, comprising a stick-shaped rod (15),
wherein a hole (16) is provided over a predetermined length extending along a longitudinal direction of the rod (15), from an end face of the rod (15), and
the portion of the rod (15) in which the hole (16) is provided is the cylindrical member (10').

8. The wiping tip according to any one of claims 1 and 3 to 7, wherein one end thereof comprises the cylindrical member (10) provided with the cotton-like wiping member (20), and another end thereof comprises a handle (30).

9. The wiping tip according to any one of claims 1 to 8, wherein the thin film portion (21) is configured to be penetrated by the male member (911) and folded into the cylindrical member (10).

10. The wiping tip according to any one of claims 1 to 9, wherein an outer diameter (D20) of the outer circumferential portion (23) is larger than an inner diameter of the female thread (915).

11. The wiping tip according to any one of claims 1 to 10, wherein an outer diameter (D10) of the cylindrical member (10) is equal to or smaller than an inner diameter of the female thread (915).

12. The wiping tip according to any one of claims 1 to 11, wherein an inner diameter (D11) of the cylindrical member (10) is equal to or larger than a maximum outer diameter of an outer circumferential face (912) of the male member (911).

## Patentansprüche

1. Wischspitze (1; 1'; 2; 3; 4; 5) zur Verwendung, um einen Steckverbinder (910) sauber zu wischen, wobei der Steckverbinder beinhaltet: ein zylindrisches Steckelement (911); einen Außenzylinder (913), der das Steckverbinder (911) umgibt; und ein Innengewinde (915), das an einer Innenumfangsfläche des Außenzylinders (913), die dem Steckelement (911) zugewandt ist, gebildet ist, wobei die Wischspitze (1; 1'; 2; 3; 4; 5) umfasst: ein hohles zylindrisches Element (10); und **gekennzeichnet durch** ein watteartiges Wischelement (20), das an dem zylindrischen Element (10) angeordnet ist,
wobei das watteartige Wischelement (20) einen Dünnfilmabschnitt (21), der eine Öffnung (11) an einem Ende des zylindrischen Elements (10) bedeckt, und einen Außenumfangsabschnitt (23) darstellt, der sich aus dem Dünnfilmabschnitt (21) erstreckt und eine Außenumfangsfläche des zylindrischen Elements (10) über eine vorbestimmte Länge (L20) von dem Ende aus bedeckt;
wobei der Dünnfilmabschnitt (21) von dem Steckelement (911) des Steckverbinders (910) durchdringbar ist, sodass das Steckelement (911) im Gebrauch über die Öffnung (11) des zylindrischen Elements (10) in das zylindrische Element (10) eintritt und das zylindrische Element (10) in einen Spalt (916) zwischen dem Steckelement (911) und dem Außenzylinder (913) eingeführt wird.

2. Wischspitze nach Anspruch 1, wobei das watteartige Wischelement (20) an beiden Enden des zylindrischen Elements (10) angeordnet ist.

3. Wischspitze nach Anspruch 1, wobei das watteartige Wischelement (20) nur an einem Ende des zylindrischen Elements (10) angeordnet ist.

4. Wischspitze nach einem der Ansprüche 1 bis 3, wobei das watteartige Wischelement (20) mit einer Reinigungslösung getränkt ist.

5. Wischspitze nach einem der Ansprüche 1 bis 4, wobei durch den Dünnfilmabschnitt (21) hindurch ein Schlitz (22) gebildet ist.

6. Wischspitze nach einem der Ansprüche 1 bis 5, wobei ein watteartiges Füllelement (25) in dem zylindrischen Element (10) platziert ist.

7. Wischspitze nach einem der Ansprüche 1 und 3 bis 6, die eine stabförmige Stange (15) umfasst,
wobei ein Loch (16) über eine vorbestimmte Länge bereitgestellt ist, das sich entlang einer Längsrichtung der Stange (15) aus einer Endfläche der Stange (15) erstreckt, und
der Abschnitt der Stange (15), in dem das Loch (16) bereitgestellt ist, das zylindrische Element (10') ist.

8. Wischspitze nach einem der Ansprüche 1 und 3 bis 7, wobei ein Ende davon das zylindrische Element (10) umfasst, das mit dem watteartigen Wischelement (20) bereitgestellt ist, und ein anderes Ende davon einen Griff (30) umfasst.

9. Wischspitze nach einem der Ansprüche 1 bis 8, wobei der Dünnfilmabschnitt (21) konfiguriert ist, um von dem Steckelement (911) durchdrungen, und in das zylindrische Element (10) gefaltet zu werden.

10. Wischspitze nach einem der Ansprüche 1 bis 9, wobei ein Außendurchmesser (D20) des Außenumfangsabschnitts (23) größer ist als ein Innendurchmesser des Innengewindes (915).

11. Wischspitze nach einem der Ansprüche 1 bis 10, wobei ein Außendurchmesser (D10) des zylindrischen Elements (10) gleich oder kleiner als ein Innendurchmesser des Innengewindes (915) ist.

12. Wischspitze nach einem der Ansprüche 1 bis 11, wobei ein Innendurchmesser (D11) des zylindrischen Elements (10) gleich oder größer als ein maximaler Außendurchmesser einer Außenumfangsfläche (912) des Steckelements (911) ist.

## Revendications

1. Embout d'essuyage (1; l' ; 2 ; 3 ; 4 ; 5) destiné à être utilisé pour nettoyer un raccord mâle (910), le raccord mâle incluant : un élément mâle cylindrique (911) ; un cylindre extérieur (913) entourant l'élément mâle (911) ; et un filetage femelle (915) formé sur une face circonférentielle intérieure du cylindre extérieur (913) faisant face à l'élément mâle (911), l'embout d'essuyage (1; 1' ; 2 ; 3 ; 4 ; 5) comprenant : un élément cylindrique creux (10) ; et **caractérisé par** un élément d'essuyage de type coton (20) agencé sur l'élément cylindrique (10),
dans lequel l'élément d'essuyage de type coton (20) constitue une partie de film mince (21) recouvrant une ouverture (11) sur une extrémité de l'élément cylindrique (10), et une partie circonférentielle extérieure (23) s'étendant à partir de la partie de film mince (21) et recouvrant une face circonférentielle extérieure de l'élément cylindrique (10) sur une longueur prédéterminée (L20) à partir de l'extrémité ;
dans lequel la partie de film mince (21) permet la pénétration de l'élément mâle (911) du raccord mâle (910) de telle sorte que, lors de l'utilisation, l'élément mâle (911) pénètre dans l'élément cylindrique (10) via l'ouverture (11) de l'élément cylindrique (10), et l'élément cylindrique (10) est inséré dans un espace (916) entre l'élément mâle (911) et le cylindre extérieur (913).

2. Embout d'essuyage selon la revendication 1, dans lequel l'élément d'essuyage de type coton (20) est agencé aux deux extrémités de l'élément cylindrique (10).

3. Embout d'essuyage selon la revendication 1, dans lequel l'élément d'essuyage de type coton (20) est agencé à une seule extrémité de l'élément cylindrique (10).

4. Embout d'essuyage selon l'une quelconque des revendications 1 à 3, dans lequel l'élément d'essuyage de type coton (20) est imprégné d'une solution de nettoyage.

5. Embout d'essuyage selon l'une quelconque des revendications 1 à 4, dans lequel une fente (22) est formée à travers la partie de film mince (21).

6. Embout d'essuyage selon l'une quelconque des revendications 1 à 5, dans lequel un élément de remplissage de type coton (25) est placé dans l'élément cylindrique (10).

7. Embout d'essuyage selon l'une quelconque des revendications 1 et 3 à 6, comprenant une tige en forme de bâton (15),
dans lequel un trou (16) est ménagé sur une longueur prédéterminée s'étendant le long d'une direction longitudinale de la tige (15), à partir d'une face d'extrémité de la tige (15), et
la partie de la tige (15) dans laquelle le trou (16) est ménagé est l'élément cylindrique (10').

8. Embout d'essuyage selon l'une quelconque des revendications 1 et 3 à 7, dans lequel une extrémité de celui-ci comprend l'élément cylindrique (10) doté de l'élément d'essuyage de type coton (20), et une autre extrémité de celui-ci comprend un manche (30).

9. Embout d'essuyage selon l'une quelconque des revendications 1 à 8, dans lequel la partie de film mince (21) est configurée pour être pénétrée par l'élément mâle (911) et pliée dans l'élément cylindrique (10).

10. Embout d'essuyage selon l'une quelconque des revendications 1 à 9, dans lequel un diamètre extérieur (D20) de la partie circonférentielle extérieure (23) est plus grand qu'un diamètre intérieur du filetage femelle (915).

11. Embout d'essuyage selon l'une quelconque des revendications 1 à 10, dans lequel un diamètre extérieur (D10) de l'élément cylindrique (10) est égal ou inférieur à un diamètre intérieur du filetage femelle (915).

12. Embout d'essuyage selon l'une quelconque des revendications 1 à 11, dans lequel un diamètre intérieur (D11) de l'élément cylindrique (10) est égal ou supérieur à un diamètre extérieur maximal d'une face circonférentielle extérieure (912) de l'élément mâle (911).
